# EUROPEAN PATENT APPLICATION

(11) **EP 4 332 988 A1**
(43) Date of publication of application: **06.03.2024**
(21) Application number: 22382804.7
(22) Date of filing: 29.08.2022
(51) Int. Cl.: G16H 50/30, G06N 10/00, G16H 10/60, G16H 50/20

(54) **MONITORIZATION AND FORECAST OF PAIN AND ILLNESS IN PATIENTS WITH QUANTUM COMPUTING**

(71) Applicant: Multiverse Computing S.L., 20014 Donostia-San Sebastian (ES)
(72) Inventor: Hernández, Rodrigo, 20014 Donostia-San Sebastian (ES); Orús, Román, 20014 Donostia-San Sebastian (ES)
(74) Representative: Balder IP Law, S.L.

(57) **Abstract**

A method comprising: digitally introducing data associated with at least one patient into a quantum classifier for classifying patients such that patients having a predetermined medical condition or being in risk of having the predetermined medical condition in a predetermined time span are classified into a predetermined risk group, the data comprising biometrics of the at least one patient; digitally commanding a quantum device or system to run the quantum classifier to classify the at least one patient; and digitally determining an action to be taken with respect to the at least one patient when the at least one patient has been classified into the predetermined risk group. Also, devices, systems and computer program products adapted to carry out the method.

## Description

### TECHNICAL FIELD

The present disclosure relates to the technical field of hospitals. More particularly, the present disclosure relates to the processing of data of patients for monitoring their health and forecasting possible immediate worsening of the condition of the patients with quantum computing.

### BACKGROUND

Every year millions of people worldwide require medical attention in hospitals. Even though they are patients that might stay for hours, days or weeks in the hospitals, oftentimes there are too many patients that the medical staff must monitor, and doctors and nurse are not able to devote the time needed for thoroughly monitoring the condition of the patient and inferring the possible health problems the patient has or might have in a short time period as a result of their pathologies. In turn, this causes that the patients are not adequately looked after and their medical condition worsens, sometimes fatally, because they are not treated in time.

The intensive care unit, ICU, of the hospitals is typically the area of the hospital where the patients in worst condition are looked after more carefully. When the patients are e.g. in pain, suffering a seizure, or suffering the effects of a critical illness, time is generally of the essence, hence any delay in the treatment of such patients or in the transportation to the ICU from other areas of the hospital can have fatal consequences.

It would be convenient to have a way of monitoring the patients and predicting the medical condition they might have in the near future that is both reliable and timely so that the medical staff can be better prepared and require less time to look after the patients.

### SUMMARY

A first aspect of the disclosure relates to a method comprising:
digitally introducing data associated with at least one patient into a quantum classifier for classifying patients such that patients having a predetermined medical condition or being in risk of having the predetermined medical condition in a predetermined time span are classified into a predetermined risk group, the data comprising biometrics of the at least one patient, the classifier algorithm having been trained with a training dataset comprising both historical data of biometrics of historical patients and historical data related to the medical condition of the historical patients;
digitally commanding a quantum device or system to run the quantum classifier to classify the at least one patient; and
digitally determining a first action to be taken with respect to the at least one patient when the at least one patient has been classified into the predetermined risk group, or either a second action or no action to be taken with respect to the at least one patient when the at least one patient has been classified into a predetermined group different from the predetermined risk group.

A plurality of monitored parameters of the at least one patient as provided by the plurality of sensors is to be processed by the quantum classifier to provide a classification for said patient or patients. The quantum classifier may classify the patient into the predetermined risk group that is indicative of a problematic health condition of the patient that the medical staff must monitor or react to, and also into another predetermined group, e.g. a predetermined no-risk group, that is indicative of a no problematic health condition of the patient, be it because the patient is at least somewhat healthy and, thus, does not require urgent attention from the medical staff. The quantum classifier may, in some embodiments, classify the patient into further groups, for example but without limitation, such as e.g. a predetermined patient with pain group that is indicative of an estimated level of pain suffered by the patient that might require medical attention but with lower urgency, and/or e.g. a predetermined illness group that is indicative of an existing illness suffered by the patient that might require medical attention but with lower urgency.

For the quantum classifier to provide an accurate classification, the classifier is at least trained with the two sets of historical data that relate the monitored parameters of historical patients with the medical condition of the historical patients. The classifier thus derives relationships between the different monitored parameters and the medical condition of the patients to establish e.g. coefficients and/or weights in the classifying algorithm for classifying patients into one group or another.

The use of a quantum classifier in a quantum device or system makes the classification of the patient possible in a short time span, e.g. less than 1 minute, 30 seconds, 10 seconds, or even less, and with richer and more complex classifying algorithms. In this sense, it is noted that a multiplicity of parameters of the patient can be monitored with different sensors, and any increase in the accuracy of the measurements they provide, in the frequency with which the sensors provide the measurements, and/or in the number of parameters that is monitored increases the complexity of the quantum classifier and/or the number of times that the classifier will be classifying patients. The classification preferably relies on big data as an input, and for that quantum classifiers are usually more reliable.

A computing device or system conducts the method as a computer-implemented method whereby it controls the quantum classifier that, upon running in the quantum device or system, provides information indicative of the current condition of the at least one patient or the expected condition of the at least one patient, typically in a short time span of e.g. 5 minutes, 10 minutes, 30 minutes, 1 hour or more, as set by a predetermined time threshold that defines the length in time of the time span from e.g. a current time instant up until the predetermined time threshold.

The quantum device or system can be or comprise the following: a universal quantum computer and/or a quantum annealer. The universal quantum computer can be built with e.g. superconducting circuits, or ion traps, or neutral atoms, or photonic systems, as known in the art. The quantum annealer can be built with e.g. superconducting circuits as known in the art.

In some embodiments, the method further comprises:
after the at least one patient has been classified by the quantum classifier, digitally introducing manually-introduced data related to the medical condition of the at least one patient into the training dataset, thereby providing an updated training dataset; and
digitally commanding the quantum device or system to run the quantum classifier to retrain itself with the updated training dataset.

The quantum classifier may be improved as new data comes in, especially data that may have been provided by medical staff monitoring and looking after patients as a result of the classification made by the quantum classifier of the patients within the premises. This means that a feedback loop can be provided whereby the quantum classifier is retrained one or, preferably, multiple times as new patients are monitored and treated.

Not only does the classifier improve over time in the classification it makes, but it also makes possible to at least partially adapt the classifier to the immediate situation in e.g. the hospital in terms of the existing patients. For instance, when there is a sudden illness or pandemic affecting people, the data of the first patient or patients with such illness that are monitored for classification thereof with the present method might improve the classification of subsequent patient or patients suffering the same illness, even if the classifier was first trained with a training dataset of historical patients having other illnesses.

In some embodiments, the retraining of the quantum classifier is based on machine learning, preferably based on MLOps.

Both machine learning and MLOps further improve the retraining of the quantum classifier for it to classify further patients more precisely. In this regard, the quantum device or system may perform quantum machine learning or quantum MLOps for automatically and progressively improve the classification algorithm.

In some embodiments, the method further comprises, prior to the digital introduction of data associated with the at least one patient into the quantum classifier, digitally commanding the quantum device or system to run the quantum classifier to train itself with the training dataset.

In some embodiments, the first action to be taken comprises at least one of the following: moving the patient to an intensive care unit, administering one or more drugs to the patient, activating one or more devices for notification, and sending a notification to one or more electronic devices associated with medical staff.

The action or actions that are digitally determined as the one or ones to be taken may be made dependent on the classification only; for instance, in one hospital it may be set that the action to be taken must be to move the patient into the ICU, or activating an alarm signal in the room of the patient.

Alternatively, the actions or actions may be made dependent upon one or more inputs apart from the classification itself. For example, a distance between the room where the patient is and the ICU, the number of people already in the ICU, one or more biometrics (e.g. one or more biological measurements of one or more sensors such as, but without limitation, current heartrate, current amount of oxygen in the blood, etc.; the biological measurements may include biochemical signals such as e.g. hormones and neurotransmitters, and/or electrical signals such as e.g. voltages and currents, and/or physical signals such as e.g. pressure and temperature ), available doctors or nurses on the floor where the patient is, etc.

In some embodiments, the second action to be taken comprises at least one of the following: logging in at least one memory unit that the at least one patient is not classified into the predetermined risk group together with the biometrics; and moving the patient out from an intensive care unit.

In some embodiments, the method further comprises digitally commanding an electronic device (e.g. a controller, a digital processor, etc.) to take the first action and/or the second action.

The first action and/or the second action may be automated by way of commands provided to the electronic device that, upon running said commands, actuate one or more apparatuses for taking the action(s), thereby freeing the medical staff from doing such tasks. For example, a drug dispenser may administer drugs automatically, a bed of the patient may have its wheels moved or be moved by way of e.g. conveyor belts, etc.

In some embodiments, the historical data related to the medical condition of the historical patients comprises an indication of whether each historical patient was or had to be moved to an intensive care unit.

The medical condition of historical patients may be linked to their presence in the ICU or to their future presence in the ICU. This means that, at least for training the quantum classifier, the biometrics of the historical data are tied to the patient being at that time or at a future time (not beyond a predetermined time threshold from when the biometrics were provided) in the ICU; the presence of the patients in the ICU is decided by medical staff, thus the training dataset trains the quantum classifier based on expert, medical decisions made by doctors and nurses, which increases the likelihood of the classifier making an accurate classification.

In some embodiments, the method further comprises digitally commanding a user presenting device to cause provision of user perceptible signals representative of the digital determination. In some embodiments, the digital command is further to cause provision of user perceptible signals representative of the biometrics of the at least one patient.

Medical staff can monitor the patient and decide the course of action for the patient by reviewing the user presenting device. By way of example, the user presenting device may be a screen, loudspeakers, a virtual reality or augmented reality headset, a mobile phone, a pager, etc.

In some embodiments, the predetermined medical condition comprises at least one of the following: one or more predetermined illnesses, and an estimated pain level exceeding a predetermined pain level threshold.

For the classification of the at least one patient into the predetermined risk group, the quantum classifier attempts to derive whether the at least one patient may have the one or more predetermined illnesses that increase the risk thereof of being in a delicate health condition, and/or whether the at least one patient is experiencing pain at an excessive level as set by the predetermined pain level threshold.

The illnesses and the predetermined pain level threshold are implicitly set by way of the training dataset since the biometrics of the historical patients is tied to the historical data representative of the medical condition of the historical patients.

In some embodiments, the biometrics of the data and/or the biometrics of the historical data comprise one or more of the following: blood pressure, heartrate, blood oxygen level, body temperature, hormone signals, and neurotransmitters.

In some embodiments, the quantum classifier comprises one of the following: a quantum support vector machine, a variational quantum classifier with data reuploading, a quantum boost algorithm based on variational quantum optimization, a quantum boost algorithm based on optimization, and a quantum neural network.

In the quantum support vector machine, the data of the patients is used to determine a mathematical hyperplane that separates patients with different characteristics (e.g., risky vs non-risky). In the variational quantum classifier with data reuploading, the parameters in a quantum circuit are fine-tuned to minimize the error in the classification, similarly to quantum neural networks. In the algorithms based on quantum boost, an ensemble of weak classifiers (which could be classical) are combined with weights, so that the weights are optimized in order to minimize the overall error in the classification of the patients. All these mathematical procedures can be specifically adapted to the number of features in the datapoints, which include the biometrics of the data and/or the biometrics of the historical data about the patients.

When the quantum device or system is or comprises a universal quantum computer, the quantum classifier can be the quantum support vector machine, the variational quantum classifier with data reuploading, or the quantum boost algorithm based on variational quantum optimization as known in the art. Conversely, when the quantum device or system is or comprises a quantum annealer, the quantum classifier can be the quantum boost algorithm based on optimization.

In some embodiments, the at least one patient is classified into a predetermined group different from the predetermined risk group when the at least one patient had been previously classified into the predetermined risk group one or more times and:
a time elapsed between the previous one or more classifications into the predetermined risk group and a current classification does not exceed a predetermined classification time threshold; or
the at least one patient had been previously classified into the predetermined risk group a plurality of times, the plurality of times exceeding a predetermined number of classifications threshold.

The classification of the at least one patient into the risk group may be limited to a number of times within a predetermined time window to avoid drawing the attention of the medical staff in a continued manner. When the medical staff looks after the patient(s) following the classification thereof into the risk group, it may occur that the medical staff is not available for inputting data that indicates that the patient(s) is being looked after, hence repeated classifications of the patient into the risk group in a short time span is distracting. The continued classification also increases the risk of not noticing other fresh classifications of different patients into the risk group for e.g. the first or second time.

Alternatively, instead of introducing additional data into the quantum classifier for establishing whether the at least one patient can or cannot be classified into the predetermined risk group based on previous classifications, the quantum classifier classifies the at least one patients based on the biometrics data only, and it is the computing device or system the device or system changing the classification or the action to be taken depending on whether the at least one patient had been classified into the predetermined risk group previously. To this end, the first action to be taken may comprise reclassifying the at least one patient into a predetermined group different from the predetermined risk group, or digitally determining the second action to be taken.

In some embodiments, the method further comprises, after each digital command to run the quantum classifier, running the quantum classifier in the quantum device or system.

In some embodiments, the method further comprises taking a plurality of biological measurements of the at least one patient with a plurality of sensors.

In some embodiments, the biometrics comprise some or all biological measurements of the plurality of biological measurements.

In some embodiments, the method further comprises digitally providing the biometrics by processing some or all biological measurements of the plurality of biological measurements.

The biometrics may include raw biological measurements, and/or processed biological measurements. The biological measurements may be digitally processed to e.g. reduce noise thereof, extract information therefrom, compute one or more biological parameters of the patient or patients by combining different biological measurements, etc.

A second aspect of the disclosure relates to a device or system comprising: means adapted to execute the steps of a method according to the first aspect.

A third aspect of the disclosure relates to system comprising: a computing device comprising at least one processor and at least one memory, the at least one memory being configured, with the at least one processor, to execute the steps of a method according to the first aspect.

In some embodiments, the system further comprises one or more of the following: the plurality of sensors couplable with the patient; the quantum device or system; at least one user input device; and at least one user presenting means.

In some embodiments, the system is a hospital or a room of a hospital.

A fourth aspect of the disclosure relates to a computer program product that has instructions which, when executed by at least one computing device, cause the at least one computing device to carry out the steps of a method according to the first aspect.

In some embodiments, the computer program product is embodied on a non-transitory computer-readable medium or a computer-readable data carrier has the computer program product stored thereon.

A fifth aspect of the disclosure relates to a data carrier signal carrying a computer program product according to the fourth aspect.

Similar advantages as those set out with reference to the first aspect of the disclosure are likewise applicable to the remaining aspects of the disclosure.

### BRIEF DESCRIPTION OF THE DRAWINGS

To complete the description and in order to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:
Figure 1 shows a system in accordance with embodiments.
Figures 2 and 3 show methods in accordance with embodiments.

### DETAILED DESCRIPTION

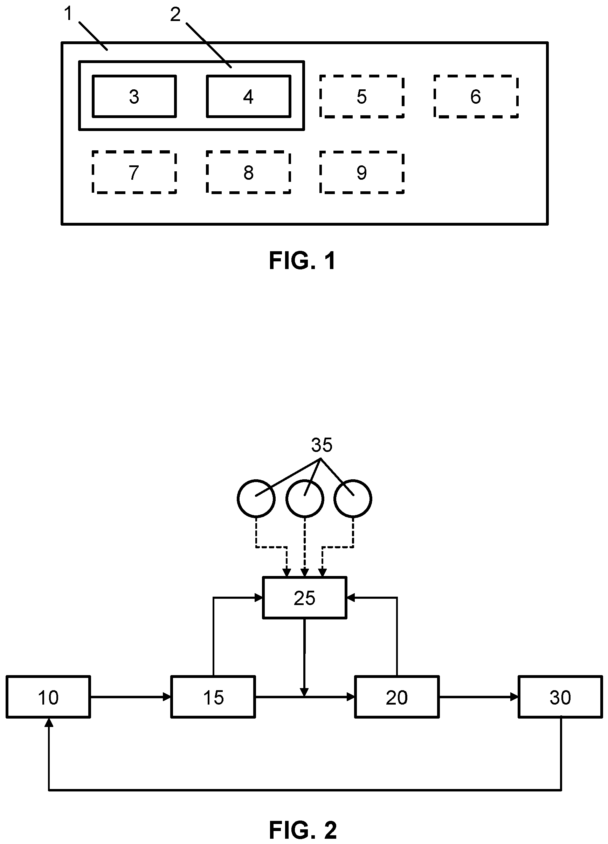

Figure 1 shows a system 1 in accordance with embodiments.

The system 1 at least comprises one or more computing devices 2, each comprising at least one processor 3 and at least one memory 4; for example, a personal computer, a laptop, a field-programmable gate array or FPGA, an application specific integrated circuit or ASIC, etc. Methods according to the present disclosure can be carried out by the system 1. To this end, the at least one memory 4 may include instructions, for example in the form of a computer program code, so that a method according to the present disclosure is carried out upon execution by the at least one processor 3.

The system 1 may comprise one or more additional devices as explained next and which are represented with dashed blocks that illustrate that the system comprises one, some or all of them in some embodiments.

The system 1 may comprise a plurality of sensors 5 configured to measure one or more biological parameters of a patient upon coupling on the patient; for example, a heartrate monitor, a respiration rate monitor, a pressure monitor, an oximeter, etc. The system 1 may comprise one or more of: a quantum device or system 6 configured to run a quantum classifier, a communications module 7 for wired or wireless communications between the one or more computing devices 2 and some other devices, including the quantum device or system 6, one or more user presenting devices 8, and one or more user input devices 9. The communications module 7 might include a converter of signals for the one or more computing devices 2 into signals for the quantum device or system 6 and vice versa, even though, sometimes, the quantum device or system 6 already includes such a converter.

Figure 2 shows a method in accordance with embodiments.

Input data 10 is provided for a quantum classifier. The input data 10 at least comprises biological measurements of a patient who is to be classified by the quantum classifier to establish its current and/or immediate health condition based on the input data 10. The biological measurements are obtained by way of multiple sensors, and preferably have a granularity of e.g. 5 milliseconds or less so that a great volume of data is considered for establishing the medical condition of the patient. Hence, the biological measurements are, in some embodiments, big data.

Whenever the quantum classifier is to be trained, for instance for initialization thereof, the input data 10 may also include a training dataset with historical data of former patients that include biometrics and medical condition data.

The input data 10 is fed to a preprocessing stage 15 whereby the input data 10 may, but not necessarily has to, be at least one of filtered, preprocessed for data extraction, converted into other type of parameters, etc. so that biometrics of the patient are provided. The input data 10 and/or the biometrics can be stored in a database, a data-lake, etc.

The biometrics are then inputted into a model 20 of the quantum classifier for monitoring and forecasting the health condition of the patient by classifying it into a particular group, i.e. classification.

Both the result of the model 20, i.e. the classification, and the biometrics from the preprocessing stage may be provided to a user presenting device 25 that may, for instance, provide a user interface to medical staff 35 for decision-making. By being provided with that information, the medical staff 35 gets to know what is the current health condition of the patient or the health condition of the patient in the near future. As part of the classification of the model 20, the user presenting device 25 may show a recommended action for the condition of the patient as derived by the model 20. By way of example, the decision-making by the medical staff 35 includes the possible movement of the patient into an ICU. The decision made, may be inputted to the model 20 for further training thereof.

A reporting stage 30 is also coupled with the model 20 for outputting metrics, including key performance indicators based on the different classifications made by the model 20, and/or for extracting information about the patients based on the different classifications made by the model 20.

Outputs from the reporting stage 30 are inputted to the input data 10 via a feedback loop for improving the model 20 of the quantum classifier.

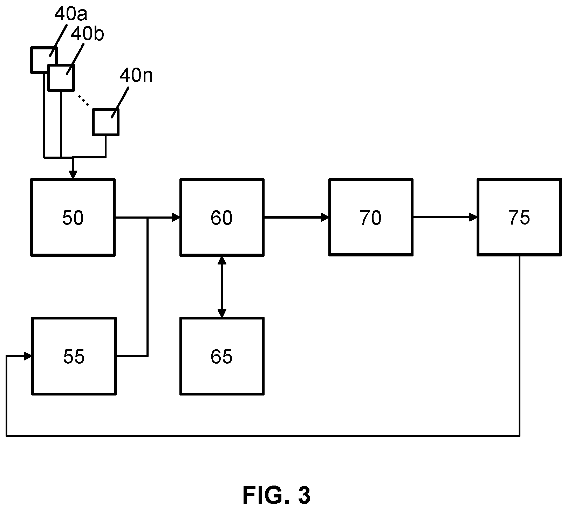

Figure 3 shows a method in accordance with embodiments.

Two different types of data 50, 55 are to be inputted into a quantum classifier for classifying the patients depending on their health condition. The first type of data 50 is biometric data resulting from biological measurements of a plurality of sensors 40a-40n. The second type of data 55 is a classification of patients preferably made by medical staff, with said classification being representative of the health condition of the patients. This second type of data can be, for instance, label data indicating whether the patients are in or were moved into an ICU. Being in or being moved into an ICU is representative of a bad health condition.

The first and second types of data 50, 55, which are streams of data, are inputted into a first module 60 that sets the quantum classifier and processes its output. The module 60, which may be part of one or more computing devices, provides a model of the quantum classifier with the inputted data 50, 55 to a quantum device or system 65 for running the model, thereby making the classification of the patients. The quantum device or system 65 then provides the classification to the first module 60. The communications between the module 60 and the quantum device or system 65 are preferably made via a secure gateway; it is noted that the quantum device or system 65 can be remote from the premises where the first module 60 is, for instance, in a datacenter that has quantum devices or systems 65 that can be used on demand upon request, e.g. via an Application Programming Interface.

The first module 60 then passes the classification and, preferably, the streams of data of the first and second types of data 50, 55, to a second module 70 that processes the received data to determine an action to be taken with respect to the patient as classified by the quantum classifier like, for instance, whether the patient shall be moved into an ICU.

The second module 70 passes the action and, preferably, all the data it received from the first module 60 to a third module 75 that provides one or more user perceptible signals that provide the information to medical staff. The medical staff can then proceed to take the action as recommended by the second module 70 or take a different action. The medical staff may then input into the third module 75 what is its medical opinion on the health condition of the patient, which may be inputted in the form of the action to be taken (e.g. patient moved to the ICU), so that this medical opinion is provided to the second type of data 55 via a feedback loop for retraining the quantum classifier and, thus, improve the classification it makes.

In this text, the term "includes", "comprises" and its derivations (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A method comprising:
digitally introducing data associated with at least one patient into a quantum classifier for classifying patients such that patients having a predetermined medical condition or being in risk of having the predetermined medical condition in a predetermined time span are classified into a predetermined risk group, the data comprising biometrics of the at least one patient, the classifier algorithm having been trained with a training dataset comprising both historical data of biometrics of historical patients and historical data related to the medical condition of the historical patients;
digitally commanding a quantum device or system to run the quantum classifier to classify the at least one patient; and
digitally determining a first action to be taken with respect to the at least one patient when the at least one patient has been classified into the predetermined risk group, or either a second action or no action to be taken with respect to the at least one patient when the at least one patient has been classified into a predetermined group different from the predetermined risk group.

2. The method of claim 1, further comprising:
after the at least one patient has been classified by the quantum classifier, digitally introducing manually-introduced data related to the medical condition of the at least one patient into the training dataset, thereby providing an updated training dataset; and
digitally commanding the quantum device or system to run the quantum classifier to retrain itself with the updated training dataset.

3. The method of claim 2, wherein the retraining of the quantum classifier is based on machine learning, preferably based on MLOps.

4. The method of any one of the preceding claims, further comprising, prior to the digital introduction of data associated with the at least one patient into the quantum classifier, digitally commanding the quantum device or system to run the quantum classifier to train itself with the training dataset.

5. The method of any one of the preceding claims, wherein the first action to be taken comprises at least one of the following: moving the patient to an intensive care unit, administering one or more drugs to the patient, activating one or more devices for notification, and sending a notification to one or more electronic devices associated with medical staff.

6. The method of any one of the preceding claims, wherein the historical data related to the medical condition of the historical patients comprises an indication of whether each historical patient was or had to be moved to an intensive care unit.

7. The method of any one of the preceding claims, further comprising digitally commanding a user presenting device to cause presentation of the digital determination.

8. The method of any one of the preceding claims, wherein the predetermined medical condition comprises at least one of the following: one or more predetermined illnesses, and an estimated pain level exceeding a predetermined pain level threshold.

9. The method of any one of the preceding claims, wherein the biometrics of the data and/or the biometrics of the historical data comprise one or more of the following: blood pressure, heartrate, blood oxygen level, body temperature, hormone signals, and neurotransmitters.

10. The method of any one of the preceding claims, wherein the quantum classifier comprises one of the following: a quantum support vector machine, a variational quantum classifier with data reuploading, a quantum boost algorithm based on variational quantum optimization, and a quantum boost algorithm based on optimization.

11. The method of any one of the preceding claims, wherein the at least one patient is classified into a predetermined group different from the predetermined risk group when the at least one patient had been previously classified into the predetermined risk group one or more times and:
a time elapsed between the previous one or more classifications into the predetermined risk group and a current classification does not exceed a predetermined classification time threshold; or
the at least one patient had been previously classified into the predetermined risk group a plurality of times, the plurality of times exceeding a predetermined number of classifications threshold.

12. The method of any one of the preceding claims, further comprising, after each digital command to run the quantum classifier, running the quantum classifier in the quantum device or system.

13. A device or system comprising: means adapted to execute the steps of a method according to any one of the preceding claims.

14. A system comprising:
a computing device comprising at least one processor and at least one memory, the at least one memory being configured, with the at least one processor, to execute the steps of a method according to any one of claims 1-11; and
one or more of: the plurality of sensors couplable with the patient; the quantum device or system; at least one user input device; and at least one user presenting means.

15. A computer program product that has instructions which, when the program is executed by at least one computing device, cause the at least one computing device to carry out the steps of a method according to any one of claims 1-11.
